# EUROPÄISCHE PATENTSCHRIFT

(11) **EP 3 548 463 B1**
(45) Veröffentlichungstag und Bekanntmachung des Hinweises auf die Patenterteilung: **14.10.2020**
(21) Anmeldenummer: 17801486.6
(22) Anmeldetag: 28.11.2017
(51) Int. Cl.: C07D 319/08, C07C 37/00, C07C 37/20, C07C 303/28

(54) **VERFAHREN ZUR HERSTELLUNG VON 3-SUBSTITUIERTEN 2-VINYLPHENYLSULFONATEN**
METHOD FOR THE PREPARATION OF 3-SUBSTITUTED 2-2-VINYLPHENYLSULFONATES
PROCÉDÉ DE FABRICATION DE 2-SULFONATES DE VINYLPHÉNYL 3-SUBSTITUÉS

(30) Priorität: 05.12.2016 EP 16202158
(43) Veröffentlichungstag der Anmeldung: 09.10.2019
(73) Patentinhaber: Bayer CropScience Aktiengesellschaft, 40789 Monheim am Rhein (DE)
(72) Erfinder: GALLENKAMP, Daniel, 42113 Wuppertal (DE); FORD, Mark, James, 65207 Wiesbaden-Breckenheim (DE); BROHM, Dirk, 40822 Mettmann (DE); ERVER, Florian, 65195 Wiesbaden (DE)
(74) Vertreter: BIP Patents
(86) Internationale Anmeldenummer: PCT/EP2017/080624
(87) Internationale Veröffentlichungsnummer: WO 2018/104105

(56) Entgegenhaltungen:
- WO-A1-2011/076699
- WO-A1-2015/189114
- WO-A1-2016/139161
- US-A- 5 424 460
- YAMAGUCHI M ET AL: "ORTHO-VINYLATION REACTION OF PHENOLS WITH ETHYNE", THE JOURNAL OF ORGANIC CHEMISTRY, AMERICAN CHEMICAL SOCIETY ETC.|, Bd. 63, Nr. 21, 1. Januar 1998 (1998-01-01), Seiten 7298-7305, XP002220698, ISSN: 0022-3263, DOI: 10.1021/JO980785F

## Beschreibung

Die vorliegende Erfindung betrifft ein Verfahren zur Herstellung von 3-substituierten 2-Vinylphenylsulfonaten, insbesondere 3-Chloro-2-vinylphenylsulfonaten.

3-substituierte 2-Vinylphenylsulfonate sind wichtige Zwischenstufen für die Herstellung agrochemischer Wirkstoffe (siehe z.B. WO 2011/076699 oder WO 2014/206896).

Die Herstellung der 3-substituierten 2-Vinylphenylsulfonate erfolgt typischerweise durch Reaktion der entsprechenden 3-substituierten 2-Vinylphenole mit einem Aryl- oder Alkylsulfochlorid. So ist beispielsweise die Herstellung von 3-Chloro-2-vinylphenol via Tetrachlorocyclohexanon und Trichloro-1,3,3-vinyl-2-oxa-7-bicyclo-[4.1.0]heptan als Zwischenstufen aus EP 0511036 B1 bekannt. Nachteile des Verfahrens sind eine geringe Gesamtausbeute sowie eine geringe Atomökonomie.

In WO 2016/139161 wird die Herstellung von 3-Chloro-2-vinylphenol in einem zweistufigen Verfahren durch Umsetzung von 2-Chloro-6-hydroxybenzaldehyd mit MeMgBr zu 3-Chlor-2-(1-hydroxyethyl)phenol und anschließende Elimination von Wasser beschrieben. Das 2-Chloro-6-hydroxybenzaldehyd wird aus 3-Chlor-2-(dichloromethyl)phenyltrichloroacetat hergestellt. Im Hinblick auf die Atomökonomie ist jedoch auch dieses Verfahren noch verbesserungswürdig. Ein alternatives Verfahren zur Herstellung von 2-Chloro-6-hydroxybenzaldehyd ist aus WO 2012/087229 bekannt. Aufgrund der verwendeten Reagenzien, wie beispielsweise DMSO als für den technischen Gebrauch ungeeignetes Lösungsmittel, und der geringen Ausbeute ist dieses Verfahren auch verbesserungswürdig.

Aufgabe der vorliegenden Erfindung war daher, ein verbessertes Verfahren zur Herstellung von 3-substituierten 2-Vinylphenylsulfonaten bereitzustellen. Das Verfahren sollte es erlauben, das gewünschte Produkt in hoher Ausbeute mit wenigen Stufen und Aufreinigungsschritten möglichst atomökonomisch herzustellen.

Diese Aufgabe wurde gelöst durch ein Verfahren zur Herstellung von 3-substituierten 2-Vinylphenylsulfonaten der Formel (I): 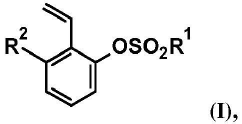 in der
R¹ für C₁-C₆-Alkyl, Phenyl, 4-Methylphenyl oder Benzyl steht, und
R² für Halogen oder Methyl steht,
dadurch gekennzeichnet, dass
(a) eine Verbindung der Formel (**II**) 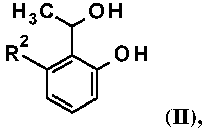 mit einem Aktivator ausgewählt aus der Gruppe bestehend aus Thionylchlorid, Phosgen, Diphosgen, Triphosgen, Thiophosgen und Chlorameisensäureestern,
   in Gegenwart einer Base zu einer Verbindung der Formel (**III**) 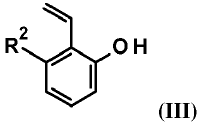 umgesetzt wird, und
(b) die Verbindung der Formel (**III**) in Gegenwart einer Base mit einer Verbindung der Formel (**IV**)

   R¹-SO₂-R³ (**IV**),

   wobei R³ für F, Cl, Br oder OSO₂R¹ steht und R¹ wie in Formel (I) definiert ist,
   zum 3-substiuierten 2-Vinylphenylsulfonat der Formel (**I**) umgesetzt wird.

Es wurde überraschend gefunden, dass 3-substituierte 2-Vinylphenylsulfonate mit Hilfe eines Aktivators, wie z.B. Phosgen oder Thionylchlorid, in hoher Ausbeute und ohne die Notwendigkeit einer Zwischenisolierung von Intermediaten aus den entsprechenden 3-substituierten 2-(1-Hydroxyethyl)-phenolen gewonnen werden können.

Gegenüber dem aus WO 2016/139161 bekannten Verfahren hat das erfindungsgemäße Verfahren den Vorteil, dass (i) eine geringere Menge an Verbindung der Formel (**IV**) eingesetzt werden muss und Thionylchlorid oder Phosgen im Vergleich zu Verbindungen der Formel (**IV**) deutlich preiswerter sind, (ii) als Abfallprodukte in Schritt (**a**) nur ein Äquivalent gasförmige Stoffe (SO₂, CO₂) entstehen anstelle von einem Äquivalent Methansulfonsäure und (iii) eine verbesserte Ausbeute erzielt wird. In einer bevorzugten Variante des erfindungsgemäßen Verfahrens kann außerdem die Base recycelt werden. Aufgrund dieser Vorteile ist das erfindungsgemäße Verfahren für einen Einsatz im technischen Maßstab besonders geeignet.

Vorzugsweise wird das erfindungsgemäße Verfahren als Eintopfverfahren ausgeführt. Erfindungsgemäß bedeutet dies, dass die Schritte (**a**) und (**b**) ohne Isolierung des Intermediats (**III**) durchgeführt werden.

Das erfindungsgemäße Verfahren ist in Schema 1 dargestellt.

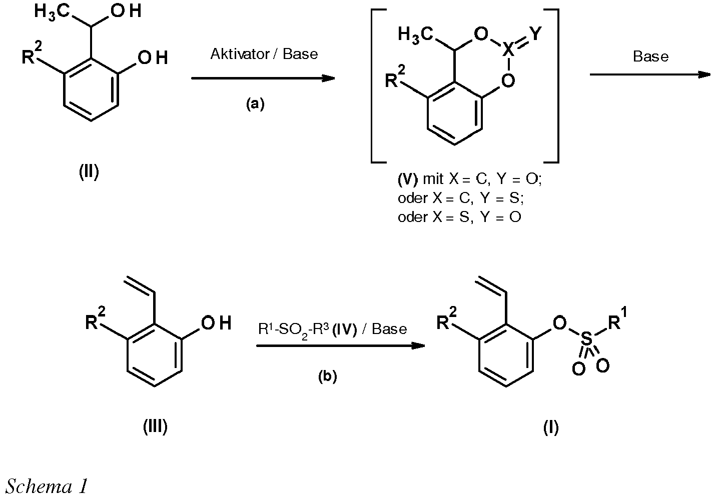

Es wurde gefunden, dass die Umsetzung der Verbindung der Formel (**II**) in Gegenwart des Aktivators und der Base zur Verbindung der Formel (**III**) über das zyklische Intermediat (**V**) verläuft. Aus dem zyklischen Intermediat (**V**) entsteht anschließend unter Eliminierung von O=X=Y die Verbindung der Formel (**III**).

Erfindungsgemäß wird die Verbindung der Formel (**III**) anschließend in Gegenwart einer Base mit einer Verbindung der Formel (**IV**) zum 3-substiuierten 2-Vinylphenylsulfonat der Formel (**I**) umgesetzt. Verbindungen der Formel (**IV**) sind kommerziell erhältlich.

*Bevorzugt* sind in dem erfindungsgemäßen Verfahren R¹ bis R³ wie folgt definiert:
- R¹: steht für Methyl, Ethyl, n-Propyl, i-Propyl, n-Butyl, Phenyl, 4-Methylphenyl oder Benzyl;
- R²: steht für Cl, F, Br, I oder Methyl; und
- R³: steht für F, Cl oder OSO₂R¹.

*Besonders bevorzugt* sind in dem erfindungsgemäßen Verfahren R¹ bis R³ wie folgt definiert:
- R¹: steht für Methyl, Ethyl, n-Propyl, Phenyl oder 4-Methylphenyl;

- R²: steht für Cl oder Br; und
- R³: steht für F, Cl oder OSO₂R¹.

*Ganz besonders bevorzugt* sind in dem erfindungsgemäßen Verfahren R¹ bis R³ wie folgt definiert:
- R¹: steht für Methyl oder 4-Methylphenyl, insbesondere für Methyl;
- R²: steht für Cl oder Br, insbesondere für Cl;
- R³: steht für F, Cl oder OSO₂R¹.

In den Schritten (**a**) und (**b**) des erfindungsgemäßen Verfahrens wird jeweils wenigstens eine Base eingesetzt. Die in den Schritten (**a**) und (**b**) eingesetzten Basen können gleich oder verschieden sein. Vorzugsweise wird in den Schritten (**a**) und (**b**) dieselbe Base eingesetzt.

Besonders bevorzugt wird das erfindungsgemäße Verfahren als Eintopfverfahren ausgeführt, wobei in den Schritten (**a**) und (**b**) dieselbe Base eingesetzt wird. Die dabei verwendete Base wird dabei zumindest teilweise, vorzugsweise vollständig, in Schritt (**a**) zugegeben.

Die Gesamtmenge an eingesetzter Base in den Schritten (**a**) und (**b**) beträgt vorzugsweise 0,9 bis 10 Äquivalente, besonders bevorzugt 1,5 bis 6,0 Äquivalente, ganz besonders bevorzugt 3,0 bis 5,0 Äquivalente, jeweils bezogen auf ein Äquivalent der Verbindung der Formel (**II**).

Als Base eignen sich ein oder mehrere organische Basen ausgewählt aus der Gruppe bestehend aus den Trialkylaminen, wie z.B. Trimethylamin, Triethylamin, Ethyldiisopropylamin, Tri-n-propylamin und Tri-n-butylamin; Alkoxid-Basen, wie z.B. Kalium-*tert*-butylat; Pyridyl-Basen, wie z.B. Pyridin, 2,6-Lutidin, 2-Picolin, 3-Picolin und 4-Picolin; und Amidinbasen, wie z.B. 1,8-Diazabicyclo[5.4.0]undec-7-en (DBU). Besonders bevorzugt ist Tri-n-butylamin.

### Allgemeine Definitionen

**Halogen** steht für F, Cl, Br oder I, bevorzugt für Cl, Br oder I, und besonders bevorzugt für Cl oder Br.

**C₁-C₆-Alkyl** steht für einen gesättigten, verzweigten oder unverzweigten Kohlenwasserstoffrest mit 1 bis 6, vorzugsweise 1 bis 3 C-Atomen, wie beispielsweise Methyl, Ethyl, n-Propyl, i-Propyl (1-Methylethyl), n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.

### Schritt (a)

In Schritt (**a**) wird die Verbindung der Formel (**II**) mit einem Aktivator in Gegenwart einer Base zur Verbindung der Formel (**III**) umgesetzt.

Erfindungsgemäß werden als Aktivator ein oder mehrere Verbindungen ausgewählt aus der Gruppe bestehend aus Thionylchlorid, Phosgen, Diphosgen, Triphosgen, Thiophosgen und den Chlorameisensäureestern eingesetzt. Als Chlorameisensäureester eignen sich Chlorameisensäure-C₁-C₆-alkylester, wie Chlorameisensäureethylester und Chlorameisensäuremethylester. Besonders bevorzugte Aktivatoren sind Thionylchlorid und Phosgen, ganz besonders bevorzugt ist Thionylchlorid.

Die eingesetzte Menge des Aktivators beträgt vorzugsweise 0.8 bis 2,5 Äquivalente, besonders bevorzugt 0,9 bis 2,0 Äquivalente, und ganz besonders bevorzugt 0.9 bis 1.5 Äquivalente, jeweils bezogen auf ein Äquivalent der Verbindung der Formel (**II**).

Die Umsetzung wird in Gegenwart einer Base und eines Lösungsmittels durchgeführt.

Geeignete Basen sind organische Basen, vorzugsweise ausgewählt aus der Gruppe bestehend aus den Trialkylaminen, wie z.B. Trimethylamin, Triethylamin, Ethyldiisopropylamin, Tri-n-propylamin oder Tri-n-butylamin; Alkoxid-Basen, wie z.B. Kalium-*tert*-butylat; Pyridyl-Basen, wie z.B. Pyridin, 2,6-Lutidin, 2-Picolin, 3-Picolin oder 4-Picolin; und Amidinbasen, wie z.B. 1,8-Diazabicyclo[5.4.0]undec-7-en (DBU). Besonders bevorzugt ist Tri-n-butylamin.

Die Menge an eingesetzter Base beträgt vorzugsweise 0,9 bis 5 Äquivalente, besonders bevorzugt 2,0 bis 3,5 Äquivalente, ganz besonders bevorzugt 3,1 Äquivalente, jeweils bezogen auf ein Äquivalent der Verbindung der Formel (**II**).

Geeignete Lösungsmittel sind aliphatische, alicyclische oder aromatische Kohlenwasserstoffe, wie beispielsweise Petrolether, n-Hexan, n-Heptan, Cyclohexan, Methylcyclohexan, Toluol, o-Xylol, m-Xylol, p-Xylol oder Decalin; halogenierte Kohlenwasserstoffe, wie beispielsweise Chlorbenzol, 1,2-Dichlorbenzol, Dichlormethan, Chloroform, Tetrachlormethan, Dichlorethan oder Trichlorethan; Ether, wie z.B. Diethylether, Diisopropylether, Methyl-tert-butylether (MTBE), Methyl-tert-amylether, 1,4-Dioxan, Tetrahydrofuran (THF), 2-Methyltetrahydrofuran (2-Me-THF), 1,2-Dimethoxyethan, 1,2-Diethoxyethan, Diglyme oder Anisol; Nitrile, wie z.B. Acetonitril, Propionitril, n-Butyronitril, Isobutyronitrile oder Benzonitril; Ester-Lösungsmittel, wie z.B. Ethylacetat; Amid-Lösungsmittel, wie z.B. N,N-Dimethylformamid (DMF) oder N,N-Dimethylacetamid (DMAc); dipolar aprotische Lösungsmittel, wie z.B. Dimethylsulfoxid (DMSO); oder Gemische dieser Lösungsmittel. Besonders bevorzugte Lösungsmittel sind Toluol, Acetonitril, MTBE, THF und 2-Me-THF, und deren Mischungen. Ganz besonders bevorzugt sind Toluol und MTBE.

Die Reaktion in Schritt (**a**) wird vorzugsweise unter Normaldruck durchgeführt, kann aber auch unter verringertem oder erhöhtem Druck durchgeführt werden.

Vorzugsweise wird eine Lösung enthaltend die Verbindung der Formel (II), die Base und das Lösungsmittel vorgelegt und unter Kühlung auf vorzugsweise -20 bis +10°C, vorzugsweise 0 bis 5°C der Aktivator zugegeben. Dabei entsteht das Intermediat (**V**).

Die anschließende Umsetzung des Intermediats (**V**) zur Verbindung der Formel (**III**) unter Eliminierung von O=X=Y erfolgt vorzugsweise bei einer Temperatur von mindestens 0°C:
Bei Einsatz einer Pyridyl-Base, wie beispielsweise 3-Picolin, in Kombination mit Thionylchlorid als Aktivator oder bei Einsatz von Thiophosgen als Aktivator beträgt die Reaktionstemperatur für die Eliminierung vorzugsweise mindestens 60 °C, besonders bevorzugt 80 - 120 °C.

Beim Einsatz einer Trialkylamin-Base, wie z.B. Tributylamin, in Kombination mit Thionylchlorid oder Phosgen als Aktivator beträgt die Reaktionstemperatur vorzugsweise 0 bis +25°C. Diese Ausführungsform ist daher besonders vorteilhaft.

Vorzugsweise wird die erhaltene Reaktionsmischung enthaltend die Verbindung der Formel (**III**) ohne Aufarbeitung in Schritt (**b**) eingesetzt.

### Schritt (b)

In Schritt (**b**) wird die Verbindung der Formel (**III**) in Gegenwart einer Base mit der Verbindung der Formel (**IV**) zum 3-substiuierten 2-Vinylphenylsulfonat der Formel (**I**) umgesetzt.

Die Menge an eingesetzter Verbindung der Formel (**IV**) beträgt vorzugsweise 0,8 bis 2,5 Äquivalente, besonders bevorzugt 0,9 bis 1,8 Äquivalente, ganz besonders bevorzugt 1,0 bis 1,5 Äquivalente, jeweils bezogen auf ein Äquivalent der Verbindung der Formel (**III**).

Die Umsetzung wird in Gegenwart einer Base und eines Lösungsmittels durchgeführt.

Geeignet sind solche Basen und Lösungsmittel, wie sie bereits für Schritt (**a**) definiert wurden. Vorzugsweise findet die Umsetzung in Gegenwart der in Schritt (**a**) eingesetzten Base und in Gegenwart des in Schritt (**a**) eingesetzten Lösungsmittels statt.

Die Menge an eingesetzter Base für Schritt (**b**) wird wie oben beschrieben vorzugsweise bereits in Schritt (**a**) zugegeben und beträgt vorzugsweise 0,9 bis 3,0 Äquivalente, besonders bevorzugt 1,0 bis 2,0 Äquivalente, ganz besonders bevorzugt 1,1 bis 1,5 Äquivalente, jeweils bezogen auf ein Äquivalent der Verbindung der Formel (**II**).

Die Reaktion in Schritt (**a**) wird vorzugsweise unter Normaldruck durchgeführt, kann aber auch unter verringertem oder erhöhtem Druck durchgeführt werden.

Die Reaktionstemperatur beträgt vorzugsweise -20 bis +100°C, besonders bevorzugt -10 bis +60°C, und ganz besonders bevorzugt 0 bis +30°C.

Vorzugsweise wird das Rohprodukt nach Ende der Reaktion zunächst mit wässriger Säure gewaschen und extrahiert, die vereinigten organischen Phasen werden abgetrennt und das Lösungsmittel wird unter reduziertem Druck entfernt. Das Produkt kann durch Umkristallisation aus beispielsweise Ethanol gereinigt werden.

In einer besonders vorteilhaften Ausführungsform des erfindungsgemäßen Verfahrens, in der Tri-n-butylamin als Base eingesetzt wird, wird das Tri-n-butylamin recycelt. Vorzugsweise werden dazu die vereinigten wässrigen Phasen durch Zugabe von Natriumhydroxid oder Kaliumhydroxid alkalisch gestellt und durch Abtrennung der sich dabei abscheidenden organischen Phase das Tri-n-butylamin zurückgewonnen. Das Tri-n-butylamin kann destillativ gereinigt werden.

### Intermediat der Formel (V)

Das erfindungsgemäße Verfahren zeichnet sich dadurch aus, dass die Umsetzung der Verbindung der Formel (**II**) in Gegenwart des Aktivators und der Base zur Verbindung der Formel (**III**) über das zyklische Intermediat (**V**) verläuft.

Ein weiterer Gegenstand der Erfindung ist daher eine Verbindung der Formel (**V**), 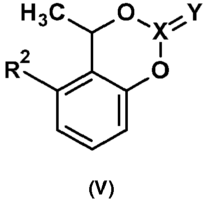 wobei X für C oder S steht, Y für S oder O steht, und X und Y nicht beide für S stehen können (d.h. X ≠ Y); und R² wie in Formel (II) definiert ist.

Besonders bevorzugt sind Verbindungen der Formel (**V**), in der X für C steht und Y für S oder O steht. Ganz besonders bevorzugt sind Verbindungen der Formel (**V**), in der X für S steht und Y für O steht.

### Herstellung der Verbindung der Formel (II)

Die Verbindung der Formel (**II**) kann durch Umsetzung einer Verbindung der Formel (**VI**) oder eines Alkali- oder Erdalkalimetallsalzes der Verbindung der Formel (**VI**) 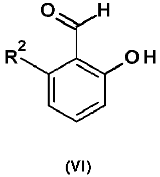 mit wenigstens einer Verbindung der Formel (**VII**)

Me-Q (**VII**)

in Gegenwart eines Lösungsmittels erhalten werden, wobei Q für Li, Na, K, MgCl, MgBr oder MgI steht, und Me für Methyl steht.

Bevorzugt steht Q für Li, MgCl, MgBr oder MgI, und besonders bevorzugt für MgCl oder MgBr.

Bei der Umsetzung der Verbindung der Formel (**VI**) mit der organometallischen Verbindung der Formel (**VII**) wird die Verbindung der Formel (**VI**) sowohl deprotoniert als auch die Verbindung der Formel (**VII**) nukleophil addiert, sofern die Verbindung der Formel (**VI**) nicht bereits als Phenolat-Anion beispielsweise in Form eines Alkali- oder Erdalkalimetallsalzes vorliegt.

Bevorzugte Alkalimetallsalze sind Salze des Natriums und Kaliums. Bevorzugte Erdalkalisalze sind Salze des Magnesiums oder Calciums.

Die Verbindung der Formel (**VII**) kann sowohl als Deprotonierungsmittel als auch als Nukleophil für die Additionsreaktion dienen.

### Variante i)

Soweit ein oder mehrere Verbindungen der Formel (**VII**) sowohl als Deprotonierungsmittel als auch als Nukleophil eingesetzt werden, beträgt die Gesamtmenge an eingesetzter Verbindung der Formel (**VII**) vorzugsweise 1,8 bis 4,0 Äquivalente, besonders bevorzugt 2,0 bis 2,5 Äquivalente, jeweils bezogen auf 1 Äquivalent der Verbindung der Formel (**VI**).

### Variante ii)

Vorzugsweise wird jedoch ein Deprotonierungsmittel eingesetzt, welches keine Verbindung der Formel (**VII**) ist. In dieser Ausführungsform wird die Verbindung der Formel (**VI**) zunächst in Gegenwart eines Lösungsmittels mit dem Deprotonierungsmittel versetzt, bevor sie mit der Verbindung der Formel (**VII**) umgesetzt wird.

Als Deprotonierungsmittel eignen sich Alkalimetallalkoholate und Alkalimetallhydroxide, vorzugsweise ausgewählt aus der Gruppe bestehend aus Natriumhydroxid, Kaliumhydroxid, Natriummethanolat, Natriumethanolat, Natrium-*tert*-butanolat, Kaliummethanolat und Kalium-*tert-*butanolat.

Soweit ein Deprotonierungsmittel verwendet wird, wird dieses vorzugsweise bei einer Reaktionstemperatur von 0 bis +100°C, besonders bevorzugt 20 bis +80°C, und ganz besonders bevorzugt 40 bis +80°C zugegeben.

Als Lösungsmittel für die Deprotonierung eignen sich Ether wie THF, Me-THF, Dioxan, MTBE oder Anisol, Alkohole wie Methanol oder Ethanol und aromatische Kohlenwasserstoffe wie Toluol, Xylol, Chlorbenzol oder 1,2-Dichlorbenzol. Besonders bevorzugt ist THF, MTBE oder Toluol in Kombination mit einer Lösung von Natriummethanolat in Methanol.

Wasser und protische Lösungsmittel wie Alkohole müssen entfernt werden bevor die deprotonierte Verbindung der Formel (**VI**) mit der metallorganischen Verbindung der Formel (**VII**) umgesetzt wird. Im Falle von Wasser wird dieses vorzugsweise durch azeotrope Destillation mit beispielsweise Toluol entfernt. Im Falle von Alkoholen wie Methanol oder Ethanol kann das Entfernen durch Destillation in Gegenwart eines höher siedenden Lösemittels wie beispielsweise Toluol erfolgen.

Durch den Einsatz des Deprotonierungsmittels kann die Menge an eingesetzter Verbindung der Formel (**VII**) reduziert werden. So werden bei Einsatz eines Deprotonierungsmittels vorzugsweise höchstens 1,8 Äquivalente, besonders bevorzugt höchstens 1,5 Äquivalente, und ganz besonders bevorzugt 0,9 bis 1,2 Äquivalente an Verbindung der Formel (**VII**), jeweils bezogen auf 1 Äquivalent der Verbindung der Formel (**VI**), verwendet.

Als Lösungsmittel für die Addition des metallorganischen Reagenzes (**VII**) eignen sich aliphatische, alicyclische oder aromatische Kohlenwasserstoffe, wie beispielsweise Petrolether, n-Hexan, n-Heptan, Cyclohexan, Methylcyclohexan, Toluol, o-Xylol, m-Xylol, p-Xylol oder Decalin; Ether, wie z.B. Diethylether, Diisopropylether, Methyl-tert-butylether (MTBE), Methyl-tert-amylether, 1,4-Dioxan, Tetrahydrofuran (THF), 2-Methyltetrahydrofuran (2-Me-THF), 1,2-Dimethoxyethan, 1,2-Diethoxyethan oder Anisol; und Gemische dieser Lösungsmittel. Besonders bevorzugte Lösungsmittel sind Toluol, o-Xylol, m-Xylol, p-Xylol, n-Hexan, Cyclohexan, Methylcyclohexan, MTBE, THF oder 2-Me-THF, und deren Mischungen. Ganz besonders bevorzugt sind THF, 2-Me-THF, MTBE, Toluol, o-Xylol, m-Xylol, p-Xylol und deren Mischungen.

### Herstellung der Verbindung der Formel (VI)

Die Verbindung der Formel (**VI**), in der R² für Cl steht, kann durch Umsetzung einer Verbindung der Formel (**VIII**) 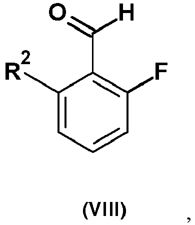 wobei R² für Cl steht,
mit einer Hydroxid-Base in Gegenwart eines Lösungsmittels erhalten werden.

Diese Synthese der Verbindung der Formel (**VI**) ist grundsätzlich aus WO 2012/087229 bekannt. Dort wird die Reaktion unter Einsatz von KOH in DMSO als Lösungsmittel beschrieben. DMSO ist jedoch ein für den technischen Gebrauch ungeeignetes Lösungsmittel und die beschriebene Ausbeute ist moderat.

Als Hydroxid-Base in dem erfindungsgemäßen Verfahren eignen sich Alkalimetallhydroxide, insbesondere Natriumhydroxid und Kaliumhydroxid. Besonders bevorzugt sind wässrige Alkalimetallhydroxidlösungen, insbesondere Kaliumhydroxidlösungen und wässrige Natriumhydroxidlösungen.

Als Lösungsmittel eignen sich Amid-Lösungsmittel, wie z.B. N,N-Dimethylformamid (DMF) und N,N-Dimethylacetamid (DMAc); Diglyme; Mischungen dieser Lösungsmittel sowie Lösungsmittelgemische von Amid-Lösungsmitteln oder Diglyme mit aromatischen Lösungsmitteln, wie z.B. Toluol, Xylol (o-Xylol, p-Xylol und/oder m-Xylol), Chlorbenzol und 1,2-Dichlorbenzol, oder Ether-Lösungsmitteln, wie THF, Me-THF und Dioxan.

Bevorzugt wird Diglyme oder ein Amid-Lösungsmittel ausgewählt aus DMF und DMAc eingesetzt. Besonders bevorzugt wird DMAc verwendet.

Ganz besonders bevorzugt wird eine Kombination aus wässriger Kaliumhydroxid-Lösung als Hydroxid-Base und N,N-Dimethylacetamid als Lösungsmittel eingesetzt.

Die erfindungsgemäß bevorzugte Synthese der Verbindung der Formel (**II**) ausgehend von der Verbindung der Formel (**VIII**) über die Verbindung der Formel (**VI**) ist in Schema 2 dargestellt, wobei R² jeweils für Cl steht:

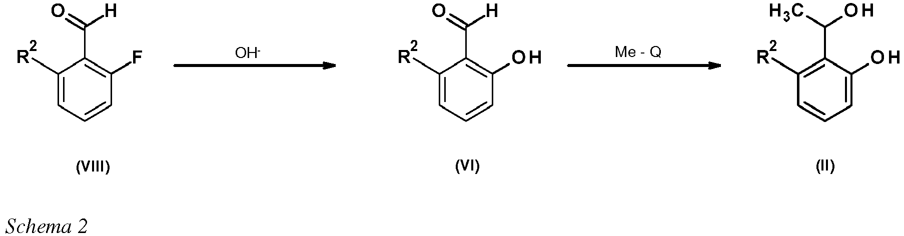

### Beispiele

Die vorliegende Erfindung wird anhand der nachfolgenden Beispiele näher erläutert, ohne die Erfindung dabei auf diese einzuschränken.

### Beispiel 1

### 2-Chlor-6-hydroxybenzaldehyd (VI, R² = Cl)

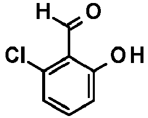

In einem 500 ml 4-Halskolben unter Stickstoff wird eine Lösung von 50.0 g (0.31 mol, 1.0 eq) 2-Chlor-6-fluorbenzaldehyd in 150 ml N,N-Dimethylacetamid bei 23 °C vorgelegt. 87.6 g (0.78 mol, 2.5 eq) 50% wässrige KOH Lösung wird bei 20 - 30 °C innerhalb von 3h zudosiert. Die Reaktion ist leicht exotherm und man erhält eine gelbe Suspension. Nach beendeter Zugabe wird 1h bei 23 °C nachgerührt. GC zeigt > 99% Umsatz. Unter Kühlung wird bei 10 - 30 °C 200 ml Wasser zugegeben und anschließend 80.0 g 37% HCl innerhalb von 10 - 20 min zudosiert (pH = 1 - 2). Es fällt ein beiger Feststoff aus.

*Wahlweise* kann dieser Feststoff durch Filtration isoliert werden. Nach Trocknung bei 20 mbar / 23 °C erhält man 43.0 g eines beige farbenen Feststoffs (88% Ausbeute).

*Alternativ* wird 150 ml Toluol zugegeben und 10 min bei 23 °C gerührt. Die wässrige Phase wird abgetrennt und nochmals mit 150 ml Toluol extrahiert. Die vereinigten organischen Phasen werden mit 150 ml 10% HCl gewaschen und am Rotationsverdampfer bei 40 °C / 50 mbar bis auf ca. 250 g eingeengt. Die Produkt Lösung wird so im nächsten Schritt verwendet (berechnete Ausbeute durch Gehaltsbestimmung mittels quant. NMR: 90%).

¹H-NMR (CDCl₃, 400 MHz) δ (ppm) = 11.91 (s, 1H), 10.42 (s, 1H), 7.42 (t, J = 8.3 Hz, 1H), 6.96 (dd, J = 8.0 Hz, 1.0 Hz, 1H), 6.90 (d, J = 8.4 Hz, 1H).

### Beispiel 2

### 3-Chlor-2-(1-hydroxyethyl)phenol (II, R² = Cl)

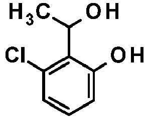

In einem 1 L 4-Halskolben unter Stickstoff wird die Produkt-Lösung aus Beispiel 1 bestehend aus 43.8 g (0.28 mol, 1.0 eq) 2-Chlor-6-hydroxybenzaldehyd in Toluol (90% ermittelte Ausbeute) vorgelegt und auf 60 °C erhitzt. 50.3 g (0.28 mol, 1.0 eq) 30% Natriummethylat Lösung in Methanol wird bei 60 °C innerhalb von 1h zudosiert und nach beendeter Zugabe 2h bei 60 °C nachgerührt. Die gelbe Suspension wird auf 23 °C abgekühlt, mit 300 ml Toluol versetzt und am Rotationsverdampfer bei 40 °C / 50 mbar eingeengt bis auf ein Restgewicht von ca. 250 g. 105.4 g (0.31 mol, 1.1 eq) 22% Methylmagnesiumchlorid Lösung in THF wird bei 20 - 30 °C innerhalb von 3h zudosiert und 1h bei 23 °C nachgerührt. HPLC zeigt vollständigen Umsatz. Unter Kühlung wird bei 10 - 20 °C innerhalb von 30min 250 ml 10% HCl zudosiert (pH = 1-2). Die wässrige Phase wird abgetrennt und mit 200 ml Toluol extrahiert. Die vereinigten organischen Phasen werden mit 100 ml 10% NaHCO3 Lösung gewaschen. Die organische Phase wird abgetrennt, mit 100 ml Wasser gewaschen und am Rotationsverdampfer bei 40 °C / 50 mbar eingeengt bis auf Restgewicht von ca. 200 g. Die Produktlösung wird so im nächsten Schritt verwendet (berechnete Ausbeute durch Gehaltsbestimmung mittels quant. NMR: 98%).

¹H-NMR (CDCl₃, 400 MHz) δ (ppm) = 8.84 (s, 1H), 7.07 (t, J = 8.0 Hz, 1H), 6.85 (dd, J = 8.0 Hz, 1.2 Hz, 1H), 6.78 (dd, J = 8.3 Hz, 1.1 Hz, 1H), 5.60 (qd, J = 6.5 Hz, 3.1 Hz, 1H), 2.65 (m, 1H), 1.57 (d, J = 6.5 Hz, 3H).

### Beispiele 3-6

Analog der Verfahrensbeschreibung des Beispiels 2 zur Herstellung der Verbindung der Formel (II) mit R² = Cl können die Verbindungen der Formel (II) der nachfolgenden Beispiele 3-6 erhalten werden:

### Beispiel 3: 3-Fluor-2-(1-hydroxyethyl)phenol (II, R² = F)

¹H-NMR (CDCl₃, 400 MHz) δ (ppm) = 8.59 (br s, 1H), 7.08 (dt, J = 8.3 Hz, 6.5 Hz, 1H), 6.65 (d, J = 8.3 Hz, 1H), 6.58 - 6.51 (m, 1H), 5.48 (q, J = 6.5 Hz, 1H), 2.69 (br s, 1H), 1.58 (d, J = 6.7 Hz, 3H).

### Beispiel 4: 2-(1-Hydroxyethyl)-3-iodphenol (II, R² = I)

¹H-NMR (CDCl₃, 400 MHz) δ (ppm) = 8.88 (br s, 1H), 7.33 (dd, J = 7.5 Hz, 1.6 Hz, 1H), 6.88 - 6.80 (m, 2H), 5.38 (q, J = 6.7 Hz, 1H), 2.80 (br s, 1H), 1.54 (d, J = 6.7 Hz, 3H).

### Beispiel 5: 3-Brom-2-(1-hydroxyethyl)phenol (II, R² = Br)

¹H-NMR (CDCl₃, 400 MHz) δ (ppm) = 9.02 (br s, 1H), 7.04 (dd, J = 8.0 Hz, 1.1 Hz, 1H), 6.99 (t, J = 8.0 Hz, 1H), 6.82 (dd, J = 8.0 Hz, 1.3 Hz, 1H), 5.53 (q, J = 6.7 Hz, 1H), 3.25 (br s, 1H), 1.56 (d, J = 6.7 Hz, 3H).

### Beispiel 6: 2-(1-Hydroxyethyl)-3-methylphenol (II, R² = Me)

¹H-NMR (CDCl₃, 400 MHz) δ (ppm) = 8.61 (br s, 1H), 7.04 (t, J = 8.0 Hz, 1H), 6.73 (d, J = 8.0 Hz, 1H), 6.65 (d, J = 7.5 Hz, 1H), 5.33 (q, J = 6.7 Hz, 1H), 2.92 (br s, 1H), 2.22 (s, 3H), 1.54 (d, J = 6.7 Hz, 3H).

### Beispiel 7

### 3-Chlor-2-vinylphenyl-methansulfonat (I, R¹ = Me, R² = Cl)

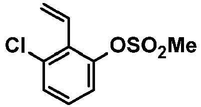

### Stufe a) 3-Chlor-2-vinylphenol (III, R² = Cl)

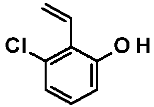

In einem 1 L 4-Halskolben unter Stickstoff werden die Produkt-Lösung aus Beispiel 2 bestehend aus 46.6 g (0.27 mol, 1.0 eq) 3-Chlor-2-(1-hydroxyethyl)phenol in Toluol (98% ermittelte Ausbeute) und 222.3 g (1.2 mol, 4.5 eq) Tributylamin vorgelegt und eine Lösung bestehend aus 35.7 g (0.30 mol, 1.1 eq) Thionylchlorid in 30 ml Toluol bei 0 - 5 °C über einen Zeitraum von 2h zudosiert. Nach beendeter Zugabe wird 1h bei 23 °C gerührt. HPLC zeigt > 98% Umsatz zum Styrol. Analytische Daten von 3-Chlor-2-vinylphenol (**III**, R² = Cl) sind wie folgt: ¹H-NMR (CDCl₃, 400 MHz) δ (ppm) = 7.08 (dd, J = 8.0, 8.0 Hz, 1H), 6.96 (d, J = 8.0 Hz, 1H), 6.84 (d, J = 8.0 Hz, 1H), 6.79 (dd, J = 12.0 Hz, 12.0 Hz, 1H), 5.74 (d, J = 12.0 Hz, 1H), 5.73 (s, 1H), 5.68 (d, J = 12.0 Hz, 1H).

Das zyklische Intermediat 5-Chlor-4-methyl-4H-1,3,2-benzodioxathiin-2-oxid (**V**, R² = Cl, X = S, Y = O) kann nicht isoliert bzw. durch diverse analytische Methoden detektiert werden. Das durch Wasser ringgeöffnete, hydrolysierte Derivat 1-(2-Chlor-6-hydroxyphenyl)ethylhydrogensulfit kann während der Reaktion mittels LC-MS in sehr geringen Mengen (< 5%) detektiert werden. Analytische Daten 1-(2-Chlor-6-hydroxyphenyl)ethylhydrogensulfit sind wie folgt: ESI neg. m/z = 235 [M-H]⁺; Retentionszeit: 1.18 min (HPLC-Säule: Phenomenex Kinetex C18, 100 mm x 2.1 mm x 1.7 µl, Eluent A: 0.1 % Ameisensäure/Wasser, Eluent B: Acetonitril, Gradient: 90/10 (0 min) → 18/82 (2.4 min) → 0/100 (2.6 min) → 0/100 (3.59 min), Fluss: 0.8 ml/min, Ofen: 40 °C, Inj.: 1.0 µl).

### Stufe b) 3-Chlor-2-vinylphenyl-methansulfonat (I, R¹ = Me, R² = Cl)

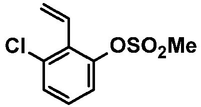

Anschließend werden bei 0 - 5 °C 36.6 g (0.32 mol, 1.2 eq) Methansulfonylchlorid innerhalb von 2h zudosiert. Nach beendeter Zugabe wird 1h bei 23 °C gerührt. HPLC zeigt > 98% Umsatz zum Methansulfonat. Das Reaktionsgemisch wird bei 0 - 10 °C innerhalb 1h zu 350 ml 10% HCl zudosiert und die wässrige Phase anschließend zweimal mit je 200 ml Toluol extrahiert. Die vereinigten organischen Phasen werden mit 150 ml 10% HCl gewaschen und am Rotationsverdampfer bei 40 °C / 50 mbar eingeengt. Der ölige Rückstand wird in 50 ml DMAc aufgenommen und bei 15 - 25 °C innerhalb von 1h zu einer Mischung aus 125 ml 37% HCl und 500 ml Wasser zudosiert. Der beige farbene Feststoff wird abgesaugt und mit 200 ml Wasser gewaschen. Nach Trocknung bei 35 °C / 20 mbar erhält man 63.7 g eines beige farbenen Feststoffs (88% Ausbeute; 78% Ausbeute über alle Stufen; 88% Reinheit nach quant. HPLC).

Durch Umkristallisation aus 30 g Ethanol erhält man 51.2 g farblose Kristalle (80% Ausbeute; 70% Ausbeute über alle Stufen; >98% Reinheit nach quant. HPLC)

¹H-NMR (CDCl₃, 400 MHz) δ (ppm) = 7.36 (dd, J = 8.0, 1.2 Hz, 1H), 7.34 (dd, J = 8.0, 1.2 Hz, 1H), 7.23 (t, J = 8.0 Hz, 1H), 6.76 (dd, J = 18.0 Hz, 11.7 Hz, 1H), 5.91 (dd, J = 18.0, 1.6 Hz, 1H), 5.73 (dd, J = 11.8, 1.4 Hz, 1H), 3.11 (s, 3H).

### Beispiel 8

### 3-Chlor-2-vinylphenyl-methansulfonat (I, R¹ = Me, R² = Cl): Variante mit isolierter Ausbeute über die letzten beiden Stufen und Recycling von Tri-n-butylamin.

### Stufe a) 3-Chlor-2-vinylphenol (III, R² = Cl)

In einem 250 ml 4-Halskolben unter Stickstoff wird eine Lösung von 10.0 g (57.9 mmol, 1.0 eq) 3-Chlor-2-(1-hydroxyethyl)phenol sowie 48.3 g Tri-n-butylamin (260.7 mmol, 4.5 eq) in 50 ml Methyl-tert-butylether vorgelegt und 7.6 g (63.7 mmol, 1.1 eq) Thionylchlorid bei 0 - 5 °C über einen Zeitraum von 1h zudosiert. Nach beendeter Zugabe wird 1h bei 23 °C gerührt. HPLC zeigt > 98% Umsatz zum Styrol. Analytische Daten von 3-Chlor-2-vinylphenol (**III**, R² = Cl) sind wie folgt: ¹H-NMR (CDCl₃, 400 MHz) δ (ppm) = 7.08 (dd, J = 8.0, 8.0 Hz, 1H), 6.96 (d, J = 8.0 Hz, 1H), 6.84 (d, J = 8.0 Hz, 1H), 6.79 (dd, J = 12.0 Hz, 12.0 Hz, 1H), 5.74 (d, J = 12.0 Hz, 1H), 5.73 (s, 1H), 5.68 (d, J = 12.0 Hz, 1H).

### Stufe b) 3-Chlor-2-vinylphenyl-methansulfonat (I, R¹ = Me, R² = Cl)

Anschließend werden bei 0 - 5 °C 8.0 g (69.5 mmol, 1.2 eq) Methansulfonylchlorid innerhalb von 1h zudosiert. Nach beendeter Zugabe wird 1h bei 23 °C gerührt. HPLC zeigt > 98% Umsatz zum Methansulfonat. 100 ml 10% HCl werden bei 10 - 15 °C zum Reaktionsgemisch zudosiert und die Phasen anschließend getrennt. Die wässrige Phase wird zweimal mit jeweils 100 ml Methyl-tert-butylether extrahiert. Die vereinigten organischen Phasen werden zweimal mit jeweils 50 ml 10% HCl gewaschen, über MgSO4 getrocknet und am Rotationsverdampfer bei 40 °C / 10 mbar eingeengt (13.9 g; Reinheit nach quant. HPLC: 83%; 86% Ausbeute). Das so erhaltene Material wird aus Ethanol/n-Heptan umkristallisiert (10.8 g; Reinheit nach quant. HPLC: 99%; 80% Ausbeute).

Die wässrige Phase wird durch Zugabe von 50% NaOH auf einen pH-Wert von 11-12 gebracht. Die sich abscheidende organische Phase wird abgetrennt und am Rotationsverdampfer bei 90 °C / 10 mbar überdestilliert. Man erhält eine farblose Flüssigkeit, die sich durch Vergleich mit Literaturdaten (1H-NMR) als Tri-n-butylamin identifizieren lässt (43.6 g; 90% Rückgewinnung).

### Beispiele 9-13

Analog zu den Verfahrensbeschreibungen der Beispiele 7 und 8 können die nachfolgend genannten Verbindungen der Formel (**III**) bzw. (**I**) erhalten werden:

### Beispiel 9: 3-Brom-2-vinylphenol (III, R² = Br):

¹H-NMR (CDCl₃, 400 MHz) δ (ppm) = 7.13 (dd, J = 7.8, 1.3 Hz, 1H), 7.00 (t, J = 7.8 Hz, 1H), 6.94 (dd, J = 8.0, 1.3 Hz, 1H), 6.74 (dd, J = 18.1 Hz, 11.4 Hz, 1H), 5.72 (dd, J = 18.1, 1.6 Hz, 1H), 5.70 (dd, J = 11.4, 1.6 Hz, 1H).

### Beispiel 10: 3-Brom-2-vinylphenyl-methansulfonat (I, R¹ = Me, R² = Br)

¹H-NMR (CDCl₃, 600 MHz) δ (ppm) = 7.56 (dd, J = 8.1, 1.1 Hz, 1H), 7.38 (d, J = 8.2 Hz, 1H), 7.16 (t, J = 8.1 Hz, 1H), 6.72 (dd, J = 17.9 Hz, 11.7 Hz, 1H), 5.84 (dd, J = 17.9, 1.3 Hz, 1H), 5.71 (dd, J = 11.7, 1.3 Hz, 1H), 3.11 (s, 3H).

### Beispiel 11: 3-Fluor-2-vinylphenyl-methansulfonat (I, R¹ = Me, R² = F)

¹H-NMR (CDCl₃, 400 MHz) δ (ppm) = 7.27 - 7.24 (m, 1H), 7.23 - 7.19 (m, 1H), 7.10 - 7.04 (m, 1H), 6.75 (dd, J = 18.0 Hz, 11.9 Hz, 1H), 6.06 (dd, J = 18.0, 1.3 Hz, 1H), 5.68 (dd, J = 11.9, 1.6 Hz, 1H), 3.15 (s, 3H).

### Beispiel 12: 3-Iod-2-vinylphenyl-methansulfonat (I, R¹ = Me, R² = I)

¹H-NMR (CDCl₃, 400 MHz) δ (ppm) = 7.84 (dd, J = 8.1 Hz, 1.1 Hz, 1H), 7.41 (dd, J = 8.1 Hz, 1.1 Hz, 1H), 7.00 (t, J = 8.0 Hz, 1H), 6.60 (dd, J = 17.8 Hz, 11.6 Hz, 1H), 5.72 (dd, J = 17.8, 1.4 Hz, 1H), 5.67 (dd, J = 11.6, 1.4 Hz, 1H), 3.11 (s, 3H).

### Beispiel 13: 3-Methyl-2-vinylphenyl-methansulfonat (I, R¹ = Me, R² = Me)

¹H-NMR (CDCl₃, 400 MHz) δ (ppm) = 7.24 (dd, J = 8.0, 1.8 Hz, 1H), 7.19 (t, J = 7.8 Hz, 1H), 7.15 (dd, J = 7.5, 1.3 Hz, 1H), 6.71 (dd, J = 17.8 Hz, 11.8 Hz, 1H), 5.66 (dd, J = 11.8, 1.8 Hz, 1H), 5.63 (dd, J = 17.8, 1.8 Hz, 1H), 3.09 (s, 3H), 2.37 (s, 3H).

### Beispiel 14

### Alternative Bedingungen für die Darstellung von 3-Chlor-2-vinylphenol (III, R² = Cl) aus 3-Chlor-2-(1-hydroxyethyl)phenol (II, R² = Cl):

### a) 3-Picolin als Base

1.0 g **3-Chlor-2-(1-hydroxyethyl)phenol** (5.8 mmol, 1.0 eq) werden in 9 ml DMAc vorgelegt und mit 2.43 g 3-Picolin (26.1 mmol, 4.5 eq) versetzt. 0.76 g Thionylchlorid (6.4 mmol, 1.1 eq) werden bei 0 - 5 °C innerhalb von 1h zudosiert und anschließend 1h bei 23 °C gerührt. HPLC zeigt >95% Umsatz zu 3-Chlor-2-[1-(3-methylpyridinium-1-yl)ethyl]-phenolat. Analytische Daten 3-Chlor-2-[1-(3-methylpyridinium-1-yl)ethyl]-phenolat sind wie folgt: ESI pos. m/z = 248 [M+H]⁺; ¹H-NMR (CDCl₃, 400 MHz) δ (ppm) = 8.88 (d, J = 6.2 Hz, 1H), 8.73 (s, 1H), 8.25 (d, J = 8.0 Hz, 1H), 7.95 (t, J = 6.2 Hz, 1H), 7.59 (dd, J = 8.4 Hz, 1.0 Hz, 1H), 7.14 (t, J = 8.3 Hz, 1H), 6.89 (dd, J = 8.0 Hz, 1.1 Hz, 1H), 6.46 (q, J = 7.0 Hz, 1H), 2.59 (s, 3H), 2.22 (d, J = 7.0 Hz, 3H).

Das Reaktionsgemisch wird anschließend auf 120 °C erhitzt und 2h bei dieser Temperatur gerührt. HPLC zeigt >85% 3-Chlor-2-vinylphenol (**III, R² = Cl**, analytische Daten: siehe Beispiele 7 und 8, Stufe a).

### b) Phosgen als Aktivator (mit Umsetzung des Intermediats (V) zur Verbindung (III) bei 110°C)

5.0 g **3-Chlor-2-(1-hydroxyethyl)phenol** (29.0 mmol, 1.0 eq) werden in 50 ml Toluol vorgelegt und mit 21.5 g Tributylamin (115.9 mmol, 4.0 eq) versetzt. Bei 0 - 5 °C werden innerhalb von 1h 5.8 g Phosgen (58.0 mmol, 2.0 eq) eingeleitet und anschließend 1h bei 23 °C gerührt. HPLC-MS zeigt >80% 5-Chlor-4-methyl-4H-1,3-benzodioxin-2-on **(V, R² = Cl, X = C, Y = O)** als Intermediat. Analytische Daten von 5-Chlor-4-methyl-4H-1,3-benzodioxin-2-on **(V, R² = Cl, X = C, Y = O)** sind wie folgt: ESI pos. m/z = 199 [M+H]⁺; Retentionszeit: 1.02 min (HPLC-Säule: Zorbax Eclipse Plus C18, 50 mm x 2.1 mm x 1.8 µl; Eluent A: 0.1 % Ameisensäure/Acetonitril; Eluent B: 0.1 % Ameisensäure/Wasser; Gradient: 10/90 47% / min → 95/5 (0.7 min); Fluss: 1 ml/min; Ofen: 55 °C; Injektion: 0.8 µl).

Das Reaktionsgemisch wird anschließend auf 110 °C erhitzt und 12h bei dieser Temperatur gerührt. HPLC zeigt >90% 3-Chlor-2-vinylphenol (**III, R² = Cl**, analytische Daten siehe Beispiele 7 und 8, Stufe a).

### c) Phosgen als Aktivator (mit Umsetzung des Intermediats (V) zur Verbindung (III) bei Raumtemperatur)

10.0 g **3-Chlor-2-(1-hydroxyethyl)phenol** (57.9 mmol, 1.0 eq) werden in 50 ml Toluol vorgelegt und mit 43 g Tributylamin (231 mmol, 4.0 eq) versetzt. Bei 0 - 6 °C werden innerhalb von 30 min 8.6 g Phosgen (86.9 mmol, 1.5 eq) eingeleitet und anschließend 1 h bei 0°C gerührt. HPLC-MS zeigt 80% 5-Chlor-4-methyl-4H-1,3-benzodioxin-2-on (**V, R² = Cl, X = C, Y = O**) als Intermediat. Analytische Daten von 5-Chlor-4-methyl-4H-1,3-benzodioxin-2-on (**V, R² = Cl, X = C, Y = O**) sind wie folgt: ESI pos. m/z = 199 [M+H]⁺; Retentionszeit: 1.02 min (HPLC-Säule: Zorbax Eclipse Plus C18, 50 mm x 2.1 mm x 1.8 µl; Eluent A: 0.1 % Ameisensäure/Acetonitril; Eluent B: 0.1 % Ameisensäure/Wasser; Gradient: 10/90 47% / min → 95/5 (0.7 min); Fluss: 1 ml/min; Ofen: 55 °C; Injektion: 0.8 µl).

Das Reaktionsgemisch wird anschließend auf Raumtemperatur gebracht und überschüssiges Phosgen durch Einleiten eines Argon Gasstroms ausgetrieben. Dann wird mit 50 ml Methanol bei Raumtemperatur versetzt und 1 h gerührt. Das LCMS zeigt >80% 3-Chlor-2-vinylphenol. Der Ansatz wird mit verdünnter NaOH auf pH 10-11 gestellt, die Phasen getrennt und die org. Phase noch 2 x mit verdünnter NaOH extrahiert. Die wässrigen Phasen werden vereinigt, mit verdünnter HCl auf pH 2 gestellt und je 3 x mit Ethylacetat extrahiert. Die vereinigte organische Phase wird über Natriumsulfat getrocknet und am Rotationsverdampfer eingeengt. Man erhält 7,9 g 3-Chlor-2-vinylphenol **(III, R² = Cl**, analytische Daten siehe Beispiele 7 und 8, Stufe a) mit einer Reinheit von 87% (Ausbeute: 76,7% d. Th.) als gelbes Öl.

### d) Thiophosgen als Aktivator (mit Isolierung und Charakterisierung des Intermediats (V))

1.0 g **3-Chlor-2-(1-hydroxyethyl)phenol** (5.8 mmol, 1.0 eq) werden in 5 ml MTBE vorgelegt und mit 2.15 g Tri-n-butylamin (11.6 mmol, 2.0 eq) versetzt. 0.76 g Thiophosgen (6.4 mmol, 1.1 eq) werden bei 0 - 5 °C innerhalb von 1h zudosiert und anschließend 1h bei 23 °C gerührt. HPLC zeigt >80% 5-Chlor-4-methyl-4H-1,3-benzodioxin-2-thion (**V, R² = Cl, X = C, Y = S**) als Intermediat. Ca. 1 ml der Reaktionslösung werden entnommen und in kleinem Maßstab durch Waschen mit 10% HCl aufgearbeitet. Die organische Phase wird anschließend am Rotationsverdampfer eingeengt. Analytische Daten 5-Chlor-4-methyl-4H-1,3-benzodioxin-2-thion (**V, R² = Cl, X = C, Y = S**) sind wie folgt: CI m/z = 215 [M+H]⁺; ¹H-NMR (CDCl₃, 400 MHz) δ (ppm) = 7.35 (t, J = 8.3 Hz, 1H), 7.27 (dd, J = 8.0 Hz, 1.1 Hz, 1H), 7.11 (dd, J = 8.3 Hz, 0.8 Hz, 1H), 5.75 (q, J = 6.7 Hz, 3.1 Hz, 1H), 1.72 (d, J = 6.7 Hz, 3H).

1.61 g Tri-*n*-butylamin (8.7 mmol, 1.5 eq) werden zum verbliebenen Reaktionsgemisch zugegeben und anschließend auf 80 °C erhitzt. HPLC zeigt >80% Umsatz zu 3-Chlor-2-vinylphenol (**III, R²** = **Cl**, analytische Daten: siehe Beispiele 7 und 8, Stufe a).

## Patentansprüche

1. Verfahren zur Herstellung eines Vinylphenylsulfonats der Formel (**I**): 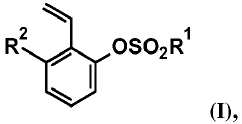 in der R¹ für C₁-C₆-Alkyl, Phenyl, 4-Methylphenyl oder Benzyl steht, und
R² für Halogen oder Methyl steht,
**dadurch gekennzeichnet, dass**
(a) eine Verbindung der Formel (**II**) 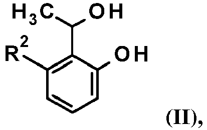 mit einem Aktivator in Gegenwart einer Base zu einer Verbindung der Formel (**III**) 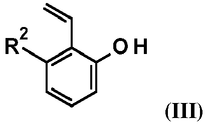 umgesetzt wird,
wobei als Aktivator ein oder mehrere Verbindungen ausgewählt aus der Gruppe bestehend aus Thionylchlorid, Phosgen, Diphosgen, Triphosgen, Thiophosgen und den Chlorameisensäureestern eingesetzt werden,
und
(b) die Verbindung der Formel (**III**) in Gegenwart einer Base mit einer Verbindung der Formel (**IV**)
R¹-SO₂-R³ **(IV),**
wobei R³ für F, Cl, Br oder OSO₂R¹ steht und R¹ wie in Formel (**I**) definiert ist, zum Vinylphenylsulfonat der Formel (**I**) umgesetzt wird.

2. Verfahren nach Anspruch 1, **dadurch gekennzeichnet, dass** die Schritte (a) und (b) in einem Eintopfverfahren durchgeführt werden.

3. Verfahren nach Anspruch 1 oder 2, **dadurch gekennzeichnet, dass** in den Schritten (a) und (b) ein oder mehrere Basen ausgewählt aus der Gruppe bestehend aus Trialkylaminen, Pyridyl-Basen, Alkoxid-Basen und Amidin-Basen eingesetzt wird.

4. Verfahren nach Anspruch 3, **dadurch gekennzeichnet, dass** als Base Tributylamin eingesetzt wird.

5. Verfahren nach einem der Ansprüche 1 bis 4, **dadurch gekennzeichnet, dass** in den Schritten (a) und (b) ein oder mehrere Lösungsmittel ausgewählt aus der Gruppe bestehend aus Petrolether, n-Hexan, n-Heptan, Cyclohexan, Methylcyclohexan, Toluol, o-Xylol, m-Xylol, p-Xylol, Decalin, Chlorbenzol, 1,2-Dichlorbenzol, Dichlormethan, Chloroform, Tetrachlormethan, Dichlorethan, Trichlorethan, Diethylether, Diisopropylether, Methyl-tert-butylether, Methyl-tert-amylether, 1,4-Dioxan, Tetrahydrofuran, 2-Methyltetrahydrofuran, 1,2-Dimethoxyethan, 1,2-Diethoxyethan, Diglyme, Anisol, Acetonitril, Propionitril, n-Butyronitril, Isobutyronitrile, Benzonitril, Ethylacetat, N,N-Dimethylformamid, N,N-Dimethylacetamid, Dimethylsulfoxid eingesetzt werden.

6. Verfahren nach einem der Ansprüche 1 bis 5, **dadurch gekennzeichnet, dass** als Lösungsmittel Toluol oder Methyl-tert-butylether eingesetzt wird.

7. Verfahren nach einem der Ansprüche 1 bis 6, **dadurch gekennzeichnet, dass**
R¹ für Methyl, Ethyl, n-Propyl, i-Propyl, n-Butyl, Phenyl, 4-Methylphenyl oder Benzyl,
R² für Cl, F, Br, I oder Methyl, und
R³ für F, Cl oder OSO₂R¹ steht.

8. Verfahren nach einem der Ansprüche 1 bis 7, **dadurch gekennzeichnet, dass** die Verbindung der Formel (**II**) durch Umsetzung einer Verbindung der Formel (**VI**)) oder eines Alkali- oder Erdalkalimetallsalzes der Verbindung der Formel (**VI**) 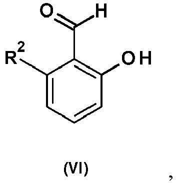 mit wenigstens einer Verbindung der Formel (**VII**)
Me-Q (**VII**)
in Gegenwart eines Lösungsmittels erhalten wird, wobei Q für Li, Na, K, MgCl, MgBr oder MgI steht.

9. Verfahren nach Anspruch 8, **dadurch gekennzeichnet, dass** die Verbindung der Formel (IV) zunächst mit einem Deprotonierungsmittel versetzt wird, bevor sie mit der Verbindung der Formel (**VII**) umgesetzt wird, wobei das Deprotonierungsmittel keine Verbindung der Formel (**VII**) ist.

10. Verfahren nach Anspruch 9, **dadurch gekennzeichnet, dass** als Deprotonierungsmittel ein Alkalimetallalkoholat und/oder Alkalimetallhydroxid eingesetzt wird.

11. Verfahren nach Anspruch 10, **dadurch gekennzeichnet, dass** das Deprotonierungsmittel ausgewählt ist aus der Gruppe bestehend aus Natriumhydroxid, Kaliumhydroxid, Natriummethanolat, Natriumethanolat, Natrium-*tert*-butanolat, Kaliummethanolat und Kalium-*tert*-butanolat.

12. Verfahren nach einem der Ansprüche 8 bis 11, **dadurch gekennzeichnet, dass** die Verbindung der Formel (**VI**) durch Umsetzung einer Verbindung der Formel (**VIII**) 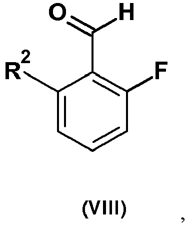 wobei R² für Methyl, I, Br oder Cl steht,
mit einer Hydroxid-Base erhalten wird.

13. Verfahren nach einem der Ansprüche 8 bis 12, **dadurch gekennzeichnet, dass** als Hydroxid-Base eine wässrige Kaliumhydroxidlösung oder eine wässrige Natriumhydroxidlösung eingesetzt wird.

14. Verfahren nach einem der Ansprüche 8 bis 13, **dadurch gekennzeichnet, dass** die Umsetzung der Verbindung der Formel (**VIII**) zur Verbindung der Formel (**VI**) in Gegenwart eines Amid-Lösungsmittels, oder eines Gemischs von Amid-Lösungsmittel oder Diglyme mit Ether-Lösungsmittel oder aromatischem Lösungsmittel durchgeführt wird.

15. Eine Verbindung der Formel (**V**), 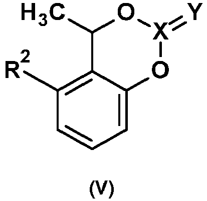 in der R² für Halogen oder Methyl steht;
X für C oder S steht, Y für S oder O steht, und wobei X und Y nicht beide für S stehen können.

## Claims

1. Method for preparing a vinylphenyl sulfonate of the formula **(I):** 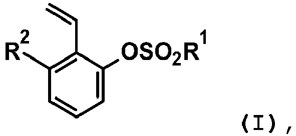 in which R¹ is C₁-C₆-alkyl, phenyl, 4-methylphenyl or benzyl, and
R² is halogen or methyl, **characterized in that**
(a) a compound of the formula **(II)** 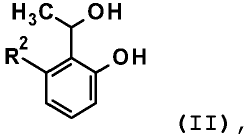 is reacted with an activator in the presence of a base to give a compound of the formula **(III)** 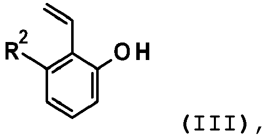 wherein one or more compounds are used as activator selected from the group consisting of thionyl chloride, phosgene, diphosgene, triphosgene, thiophosgene and chloroformic esters,
and
(b) the compound of the formula **(III)** is reacted in the presence of a base with a compound of the formula **(IV)**
R¹-SO₂-R³ **(IV),**
where R³ is F, Cl, Br or OSO₂R¹ and R¹ is as defined in formula **(I),**
to give the vinylphenyl sulfonate of the formula (I).

2. Method according to Claim 1, **characterized in that** steps (a) and (b) are carried out in a one-pot process.

3. Method according to Claim 1 or 2, **characterized in that** one or more bases selected from the group consisting of trialkylamines, pyridyl bases, alkoxide bases and amidine bases is used in steps (a) and (b).

4. Method according to Claim 3, **characterized in that** the base used is tributylamine.

5. Method according to any of Claims 1 to 4, **characterized in that** one or more solvents are used in steps (a) and (b) selected from the group consisting of petroleum ether, n-hexane, n-heptane, cyclohexane, methylcyclohexane, toluene, o-xylene, m-xylene, p-xylene, decaline, chlorobenzene, 1,2-dichlorobenzene, dichloromethane, chloroform, tetrachloromethane, dichloroethane, trichloroethane, diethyl ether, diisopropyl ether, methyl tert-butyl ether, methyl tert-amyl ether, 1,4-dioxane, tetrahydrofuran, 2-methyltetrahydrofuran, 1,2-dimethoxyethane, 1,2-diethoxyethane, diglyme, anisole, acetonitrile, propionitrile, n-butyronitrile, isobutyronitrile, benzonitrile, ethyl acetate, N,N-dimethylformamide, N,N-dimethylacetamide, dimethylsulfoxide.

6. Method according to any of Claims 1 to 5, **characterized in that** the solvent used is toluene or methyl tert-butyl ether.

7. Method according to any of Claims 1 to 6, **characterized in that**
R¹ is methyl, ethyl, n-propyl, isopropyl, n-butyl, phenyl, 4-methylphenyl or benzyl,
R² is Cl, F, Br, I or methyl, and
R³ is F, Cl or OSO₂R¹.

8. Method according to any of Claims 1 to 7, **characterized in that** the compound of the formula **(II)** is obtained by reacting a compound of the formula **(VI)** or an alkali metal salt or alkaline earth metal salt of the compound of the formula **(VI)** 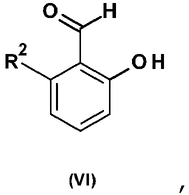 with at least one compound of the formula **(VII)**
Me-Q **(VII)**
in the presence of a solvent, where Q is Li, Na, K, MgCl, MgBr or MgI.

9. Method according to Claim 8, **characterized in that** a deprotonating agent is first added to the compound of the formula **(IV)** before it is reacted with the compound of the formula **(VII),** wherein the deprotonating agent is not a compound of the formula **(VII)**.

10. Method according to Claim 9, **characterized in that** an alkali metal alkoxide and/or alkali metal hydroxide is used as deprotonating agent.

11. Method according to Claim 10, **characterized in that** the deprotonating agent is selected from the group consisting of sodium hydroxide, potassium hydroxide, sodium methoxide, sodium ethoxide, sodium tert-butoxide, potassium methoxide and potassium tert-butoxide.

12. Method according to any of Claims 8 to 11, **characterized in that** the compound of the formula **(VI)** is obtained by reacting a compound of the formula **(VIII)** 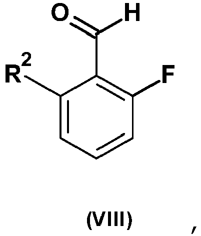 where R² is methyl, I, Br or Cl,
with a hydroxide base.

13. Method according to any of Claims 8 to 12, **characterized in that** the hydroxide base used is an aqueous potassium hydroxide solution or an aqueous sodium hydroxide solution.

14. Method according to any of Claims 8 to 13, **characterized in that** the reaction of the compound of the formula **(VIII)** to the compound of the formula **(VI)** is carried out in the presence of an amide solvent, or a mixture of amide solvent or diglyme with ether solvent or aromatic solvent.

15. Compound of the formula **(V),** 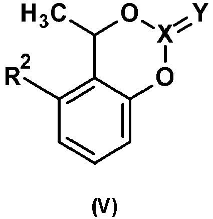 in which R² is halogen or methyl;
X is C or S, Y is S or O, and wherein X and Y cannot both be S.

## Revendications

1. Procédé pour la préparation d'un vinylphénylsulfonate de formule (I) : 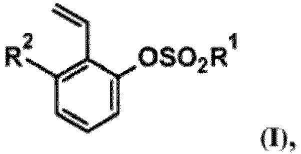 dans laquelle R¹ représente C₁-C₆-alkyle, phényle, 4-méthylphenyle ou benzyle, et
R² représente halogène ou méthyle, **caractérisé en ce que**
(a) un composé de formule (II) 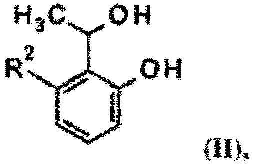 est transformé avec un activateur en présence d'une base en un composé de formule (III) 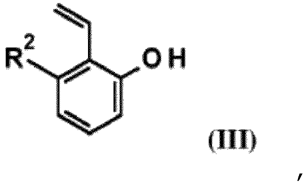 un ou plusieurs composés choisis dans le groupe constitué par le chlorure de thionyle, le phosgène, le diphosgène, le triphosgène, le thiophosgène et des esters de chloroformiate, étant utilisés en tant qu'activateur, et
(b) le composé de formule (III) est transformé en présence d'une base avec un composé de formule (IV)
R¹-SO₂-R³ **(IV),**
R³ représentant F, Cl, Br ou OSO₂R¹ et R¹ étant défini comme dans la formule (I),
pour donner le vinylphénylsulfonate de formule (I).

2. Procédé selon la revendication 1, **caractérisé en ce que** les étapes (a) et (b) sont mis en œuvre dans un procédé dans un récipient.

3. Procédé selon la revendication 1 ou 2, **caractérisé en ce que**, dans les étapes (a) et (b), une ou plusieurs bases choisies dans le groupe constitué par des trialkylamines, des bases de type pyridinyle, des bases de type alcoxyde et des bases de type amidine sont utilisées.

4. Procédé selon la revendication 3, **caractérisé en ce que** la tributylamine est utilisée en tant que base.

5. Procédé selon l'une quelconque des revendications 1 à 4, **caractérisé en ce que**, dans les étapes (a) et (b), un ou plusieurs solvants choisis dans le groupe constitué par l'éther de pétrole, le n-hexane, le n-heptane, le cyclohexane, le méthylcyclohexane, le toluène, le o-xylène, le m-xylène, le p-xylène, la décaline, le chlorobenzène, le 1,2-dichlorobenzène, le dichlorométhane, le chloroforme, le tétrachlorométhane, le dichloroéthane, le trichloroéthane, le diéthyléther, le diisopropyléther, le méthyl-tert-butyléther, le méthyl-tert-amyléther, le 1,4-dioxanne, le tétrahydrofuranne, le 2-méthyltétrahydrofuranne, le 1,2-diméthoxyéthane, le 1,2-diéthoxyéthane, le diglyme, l'anisole, l'acétonitrile, le propionitrile, le b-butyronitrile, l'isobutyronitrile, le benzonitrile, l'acétate d'éthyle, le N,N-diméthylformamide, le N,N-diméthylacétamide, le diméthylsulfoxyde, étant utilisés.

6. Procédé selon l'une quelconque des revendications 1 à 5, **caractérisé en ce que** le toluène ou le méthyl-tert-butyléther est utilisé en tant que solvant.

7. Procédé selon l'une quelconque des revendications 1 à 6, **caractérisé en ce que**
R¹ représente méthyle, éthyle, n-propyle, i-propyle, n-butyle, phényle, 4-méthylphenyle ou benzyle,
R² représente Cl, F, Br, I ou méthyle, et
R³ représente F, Cl ou OSO₂R¹.

8. Procédé selon l'une quelconque des revendications 1 à 7, **caractérisé en ce que** le composé de formule (II) est obtenu par transformation d'un composé de formule (VI) ou d'un sel de métal alcalin ou alcalino-terreux du composé de formule (VI) 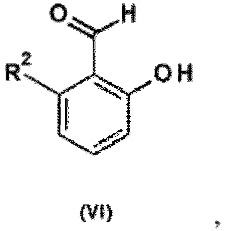 avec au moins un composé de formule (VII)
Me-Q (**VII**)
en présence d'un solvant, Q représentant Li, Na, K, MgCl, MgBr ou MgI.

9. Procédé selon la revendication 8, **caractérisé en ce que** le composé de formule (IV) est tout d'abord soumis à un agent de déprotonation, avant d'être transformé avec le composé de formule (VII), l'agent de déprotonation n'étant pas un composé de formule (VII).

10. Procédé selon la revendication 9, **caractérisé en ce qu'**un alcoolate de métal alcalin et/ou un hydroxyde de métal alcalin est utilisé en tant qu'agent de déprotonation.

11. Procédé selon la revendication 10, **caractérisé en ce que** l'agent de déprotonation est choisi dans le groupe constitué par l'hydroxyde de sodium, l'hydroxyde de potassium, le méthanolate de sodium, l'éthanolate de sodium, le tert-butanolate de sodium, le méthanolate de potassium et le tert-butanolate de potassium.

12. Procédé selon l'une quelconque des revendications 8 à 11, **caractérisé en ce que** le composé de formule (VI) est obtenu par transformation d'un composé de formule (VIII) 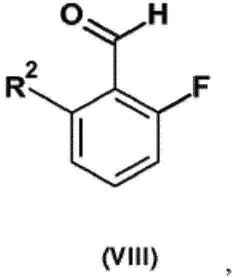 R² représentant méthyle, I, Br ou Cl,
avec une base de type hydroxyde.

13. Procédé selon l'une quelconque des revendications 8 à 12, **caractérisé en ce qu'**une solution aqueuse d'hydroxyde de potassium ou une solution aqueuse d'hydroxyde de sodium est utilisée en tant que base de type hydroxyde.

14. Procédé selon l'une quelconque des revendications 8 à 13, **caractérisé en ce que** la transformation du composé de formule (VIII) en composé de formule (VI) est mise en œuvre en présence d'un solvant de type amide, ou d'un mélange de solvant de type amide ou de diglyme avec un solvant de type ester ou un solvant aromatique.

15. Composé de formule (V), 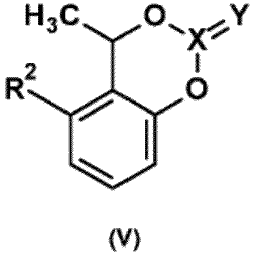 dans laquelle R² représente halogène ou méthyle ;
X représente C ou S, Y représente S ou O, et X et Y ne pouvant pas tous les deux représenter S.
